# EUROPEAN PATENT SPECIFICATION

(11) **EP 2 394 518 B1**
(45) Date of publication and mention of the grant of the patent: **22.03.2017**
(21) Application number: 10738665.8
(22) Date of filing: 03.02.2010
(51) Int. Cl.: C12N 9/98

(54) **METHOD FOR PRODUCING NOODLE, AND ENZYME PREPARATION FOR MODIFYING NOODLE**
VERFAHREN FÜR DIE HERSTELLUNG VON NUDELN SOWIE ENZYMPRÄPARATE FÜR DIE MODIFIKATION VON NUDELN
PROCÉDÉ DE PRODUCTION DE NOUILLES ET PRÉPARATION ENZYMATIQUE PERMETTANT DE MODIFIER DES NOUILLES

(30) Priority: 04.02.2009 JP 2009023298
(43) Date of publication of application: 14.12.2011
(73) Proprietor: Ajinomoto Co., Inc., Tokyo 104-8315 (JP)
(72) Inventor: YAMADA, Noriaki, Kawasaki-shi Kanagawa 210-8681 (JP)
(74) Representative: Strehl Schübel-Hopf & Partner
(86) International application number: PCT/JP2010/051908
(87) International publication number: WO 2010/090337

(56) References cited:
- WO-A1-2005/096839
- WO-A1-2008/001940
- WO-A1-2010/035858
- JP-A- 6 296 467
- JP-A- 11 137 196
- JP-A- 11 137 197
- JP-A- 61 031 954
- JP-A- 2000 060 431
- JP-A- 2006 345 870
- US-A- 4 547 280
- GESTRELIUS S ET AL: "Studies on pH-activity profiles of an immobilized two-enzyme system", BBA ENZYMOLOGY, ELSEVIER, AMSTERDAM, NL, vol. 276, no. 2, 28 August 1972 (1972-08-28), pages 339-343, XP023359649, ISSN: 0005-2744, DOI: 10.1016/0005-2744(72)90993-X [retrieved on 1972-08-28]
- LU Z -H ET AL: "Isolation, characterization and identification of lactic acid bacteria and yeasts from sour Mifen, a traditional fermented rice noodle from China", JOURNAL OF APPLIED MICROBIOLOGY, vol. 105, no. 3, September 2008 (2008-09), pages 893-903, XP9159832,
- GUJRAL HARDEEP SINGH ET AL: "Improvement of the breadmaking quality of rice flour by glucose oxidase.", FOOD RESEARCH INTERNATIONAL, vol. 37, no. 1, 2004, pages 75-81, XP002677037, ISSN: 0963-9969
- MILLER K A ET AL: "Effect of oxidation on the dynamic rheological properties of wheat flour-water doughs", CEREAL CHEMISTRY, vol. 76, no. 1, January 1999 (1999-01), pages 100-104, XP9159848, ISSN: 0009-0352

## Description

### Technical Field

The present invention relates to methods for producing noodles, and the use of an enzyme preparation for modifying noodles with α-glucosidase and glucose oxidase, or with α-glucosidase, glucose oxidase, and transglutaminase.

### Background Art

Many foods are collections of a wide variety of components such as starches, proteins, sugars, and lipids, which combine to give a texture to food. The contribution of starches and proteins to texture is particularly large, and the changes with time of starch are considered to be particularly important.

Leaving a gelatinized starch at ordinary temperature or low temperature causes the moisture to be separated and the starch hardens. This phenomenon is called retrogradation, and many studies have been made for this phenomenon. Generally, retrogradation prevention requires maintaining temperature at 80°C or greater, making moisture to 15% or less by rapid drying, or keeping alkaline conditions with a pH of 13 or greater. Known methods of preventing retrogradation generally include methods that add sugars (including glucose, fructose, and liquid sugar), soy proteins, wheat glutens, fatty acid esters, or polysaccharides (including yam and konjac) to starch-containing food. JP-A-59-2664 describes a method that adds a thickener, a surfactant, and the like. However, these methods are not sufficient as they greatly change the taste, and have unstable effects.

Methods that add enzymes are also known as a means to prevent retrogradation. For example, JP-A-58-86050 describes a method for improving cooked rice, whereby milled rice is cooked with an enzyme such as amylase, protease, and lipase mixed with a common salt and cyclodextrin. JP-A-60-199355 describes a retrogradation preventing method for cooked rice, whereby an aqueous solution of glycosylated amylase (β-amylase, glucoamylase) is sprayed over cooked rice.

There are many findings in texture improving methods for noodles, a variation of starch-containing foods. Specifically, protein materials (including vital gluten, soy protein, egg white, whole egg, and casein) and starches (including various starches, polysaccharides, and emulsifiers) are added to improve the texture of boiled noodle (JP-A-2-117353). In another method, a short-time high-temperature treatment is performed in retort sterilization to maintain texture (JP-A-2-186954). Further, methods are known that use transglutaminase to improve texture (JP-A-2-286054, JP-A-6-14733). In these methods, an inter- and intra-protein network structure is formed in the noodles by the action of transglutaminase to prevent moisture homogenization in the noodles, making it possible to maintain the preferred elastic (chewy) texture after boiling. However, the texture is uniform overall, and there is still room for improvement in obtaining a texture with a firm center, or Al dente as it is called (harder inside than outside).

WO2005/096839 describes adding α-glucosidase as an agent for improving the physical properties of starch-containing foods during the kneading of wheat, in order to obtain udon that has improved hardness and strength, and that develops a more firm center with time than that obtained without α-glucosidase. While this technique provides certain effects, there is still room for improvement in physical property immediately after boiling. Recently, there is a report that the combined use of α-glucosidase and transglutaminase in appropriate proportions improves the texture immediately after boiling, and, at the same time, maintains the improved texture over extended time periods (WO2008/001940). The effect is substantial, but is still limited in the sense that strong elasticity cannot be realized in good balance with the preferred texture.

Concerning the use of glucose oxidase for noodles, there is a report that the use of glucose oxidase in combination with amylase and glucoamylase improves chewiness (JP-A-6-296467). However, the publication does not describe using glucose oxidase with α-glucosidase or transglutaminase. There is also a report that the combined use of glucose oxidase and glucose suppresses mottling, but lowers the texture (JP-A-11-137197). In another report, glucose oxidase is described as being capable of improving shelf life (Food and Agricultural Materials Inspection Center, Examination Research Report No. 19, p. 95-101): however, there is no description concerning texture improvement. JP-A-2000-60431 discloses a method that uses transglutaminase and glucose oxidase in combination to improve a noodle texture, including the pleasant feel of a noodle through the throat, and firmness. Though this method is highly effective, there are still limits in obtaining a noodle texture that satisfies both "stickiness" and strong "elasticity".

JP 11-137196 A discloses a method for improving the qualities of a starch-containing food by using an enzyme. Gestrelius, S. et al.: "Studies on pH-activity profiles of an immobilized two-enzyme system", BBA Enzymology, Elsevier, Amsterdam, NL, vol. 276, no. 2, 28 August 1972, pages 339-343, discloses an enzyme preparation with alpha-glucosidase and glucose oxidase either immobilized on a neutral support or in a solution.

US 4 547 280 A discloses an immobilized enzyme film containing alpha-glucosidase and glucose oxidase and a solution comprising said enzymes.

JP 61 031954 A discloses an enzyme membrane having immobilized alpha-glucosidase and glucose oxidase.

JP 2006 345870 A discloses a film membrane and an electrode chemical sensor containing immobilized alpha-glucosidase and glucose oxidase.

Lu, Z.-H. et al.: "Isolation, characterization and identification of lactic acid bacteria and yeasts from sour Mifen, a traditional fermented rice noodle from China", Journal of Applied Microbiology, vol. 105, no. 3, September 2008, pages 893-903, discloses the use of a viable microorganism to assess their potential use as starter culture and their natural fermentation effect on the textural improvement of fermented rice noodles.

Gujral, Hardeep Singh et al.: "Improvement of the breadmaking quality of rice flour by glucose oxidase", Food Research International, vol. 37, no. 1, 2004, pages 75-81, discloses the use of glucose oxidase for increasing the elasticity of starch-containing food.

### Disclosure of the Invention

An object of the present invention is to provide a method for producing noodles having improved physical properties and improved taste, and the use of an enzyme preparations for modifying noodles. Specifically, the invention provides a method that improves the qualities (taste and physical properties) of noodles immediately after the production kneading material such as cereal flour, and that suppresses time-dependent quality deterioration as might occur during the producing steps, and during the course of distribution after the production. The invention also provides a method for improving the manufactuability of noodles. More specifically, the invention provides a method for producing noodles with a texture having, for example, both "stickiness" and strong "elasticity", which cannot be obtained with the sole or combined use of α-glucosidase and transglutaminase alone. Note that "stickiness" is the feel of a noodle clinging to the teeth upon chewing, and "elasticity" is the stress exerted back from chewing, specifically the extent of resilience.

The present inventor conducted intensive studies, and completed the invention based on the finding that the foregoing objects could be achieved with the use of α-glucosidase and glucose oxidase, or with α-glucosidase, glucose oxidase, and transglutaminase. Specifically, the present invention provides the following.
[1] A method for producing a noodle having improved stickiness and elasticity with α-glucosidase and glucose oxidase, wherein the α-glucosidase is used in 1.5 to 300,000 U per gram of a raw material cereal flour, and wherein the glucose oxidase is used in 0.002 to 500 U per gram of the raw material cereal flour and in 0.00006 to 3 U per unit of the α-glucosidase.
[2] The method according to [1] above, wherein the α-glucosidase is used in 3 to 15,000 U per gram of the raw material cereal flour, and wherein the glucose oxidase is used in 0.005 to 50 U per gram of the raw material cereal flour.
[3] The method according to [1] above, wherein transglutaminase is further used.
[4] The method according to [3] above, wherein the transglutaminase is used in 0.0001 to 100 U per gram of the raw material cereal flour.
[5] The method according to [3] above, wherein the transglutaminase is used in 0.0001 to 10 U per gram of the raw material cereal flour.
[6] The method according to [3] above, wherein the transglutaminase is used in 0.0000001 to 1 U per unit of the α-glucosidase.
[7] The method according to [3] above, wherein the glucose oxidase is used in 0.00006 to 3 U per unit of the α-glucosidase, and wherein the transglutaminase is added in 0.000001 to 0.1 U per unit of the α-glucosidase.
[8] The method according to [1] above, wherein the noodle is any one of pasta, udon, Chinese-style noodle, stir-fried noodle, and buckwheat noodle.
[9] The use of an enzyme preparation for modifying a noodle to have improved stickiness and elasticity, wherein the enzyme preparation contains α-glucosidase and glucose oxidase as active ingredients, wherein the content of the glucose oxidase is 0.00006 to 3 U per unit of the α-glucosidase, and wherein the α-glucosidase is used in 1.5 to 300,000 U per gram of a raw material cereal flour, and the glucose oxidase is used in 0.002 to 500 U per gram of the raw material cereal flour.
[10] The use according to [9] above, wherein the enzyme preparation further contains transglutaminase as an active ingredient.
[11] The use according to [10] above, wherein the content of the transglutaminase is 0.0000001 to 1 U per unit of the α-glucosidase.
[12] The use according to [10] above, wherein the content of the transglutaminase is 0.000001 to 0.1 U per unit of the α-glucosidase.

The present invention can improve the qualities of noodles. Specifically, the invention can produce noodles that have both "stickiness" and strong "elasticity", and can suppress the time-dependent deterioration of noodle quality.

The noodle producing method for modifying noodles according to the present invention uses α-glucosidase and glucose oxidase, or α-glucosidase, glucose oxidase, and transglutaminase.

The α-glucosidase used in the present invention is an enzyme that hydrolyzes the non-reducing terminal α-1,4-glucoside bond to produce α-glucose. Preferably, the α-glucosidase is transglucosidase that has the glycosyl transfer activity to convert the α-1,4 bond to α-1,6 bond. An example of the α-glucosidase is "Transglucosidase L Amano", commercially available from Amano Enzyme Inc.

The glucose oxidase used in the present invention is an oxidase that catalyzes the reaction producing gluconic acid and hydrogen peroxide using glucose, oxygen, and water as the substrates. The hydrogen peroxide produced by this reaction is believed to promote the formation of SS bonds (disulfide bonds) by the oxidation of the SH groups in the protein, and to form a crosslinked structure in the protein. Glucose oxidases of various origins are known, including those from microorganisms and plants. The enzyme used in the present invention may be of any origin, provided that it has the foregoing activity. Further, the enzyme may be a recombinant enzyme. The glucose oxidase of microorganism origin, commercially available under the trade name "Sumizyme PGO" from Shin-Nihon Chemical Co. is one example of such an enzyme. As in many products commercially available as mixtures of glucose oxidase and catalase preparations, the enzyme may be a mixture with other preparations, provided that it has glucose oxidase activity.

The transglutaminase used in the present invention refers to the enzyme that has the activity to catalyze the acyl transfer reaction that uses the glutamine residue and lysine residue in the protein or peptide as the donor and the receptor, respectively. Various such enzymes of different origins are known, including those frommammals, fish, and microorganisms. The enzyme used in the present invention may be of any origin, provided that the enzyme has the foregoing activity. Further, the enzyme may be a genetically recombinant enzyme. The transglutaminase of microorganism origin commercially available from Ajinomoto Co., Inc. under the trade name "Activa" TG is one example of such an enzyme.

Among a wide range of noodles available, the invention is considered particularly effective, from the standpoint of such factors as market size and needs, for noodles such as udon, pasta, buckwheat noodle, Chinese-style noodle, stir-fried noodles, and instant noodles produced through a frying step and a drying step, and for the wrapping of jiaozi and shumai.

In the production of noodles (including the wrapping of jiaozi and shumai), the raw material cereal flour such as wheat flour may be acted upon by the α-glucosidase and glucose oxidase, or by the α-glucosidase, glucose oxidase, and transglutaminase at any stage of noodle producing steps in the noodle production using these enzymes. Specifically, the enzymes may be added at the time of mixing the raw materials, or may be sprinkled after mixing. The order in which noodles are acted upon by the transglutaminase, α-glucosidase, and glucose oxidase is not particularly limited, and the enzymes may be allowed to act after one or two of these enzymes have acted first, before the remaining enzyme(s) exhibits its activity. Preferably, the three enzymes are allowed to act at the same time. The enzymes may be used in combination with other enzymes or substances (sugars such as dextrin, starch, and processed starch; seasonings such as meat extracts; proteins such as plant protein, gluten, egg white, gelatin, and casein; protein hydrolysate; protein partial hydrolysate; emulsifiers; chelating agents such as citrates and polyphosphates; reducing agents such as glutathione and cysteine; and other food additives such as alginic acid, kansui, dye, acidulant, and flavoring ingredient).

Examples of the raw material cereal flour include wheat flour, rice flour, barley flour, and rye flour. The wheat flour used may be of any variety, including, for example, hard flour, semi-hard flour, all-purpose flour, weak flour, and durum semolina flour. Further, the raw material cereal flour may be used by being mixed with a non-wheat cereal flour, such as rice flour, and starch (including processed starch).

In the present invention, the α-glucosidase is added in an appropriate range of 1.5 U or more, preferably 1.5 to 300,000 U, more preferably 3 to 15,000 U in terms of enzyme activity per gram of the raw material cereal flour. Note that 1 U (unit) of α-glucosidase enzyme activity is defined as the amount of the enzyme that produces 1 µg of glucose in 2.5 ml of a reaction liquid when 0.5 ml of an enzyme solution is allowed to act at 40°C for 60 min upon being added to a mixture containing 1 ml of 1 mM α-methyl-D-glucoside and 1 ml of 0.02 M acetate buffer (pH 5.0).

In the present invention, the glucose oxidase is added in an appropriate range of 0.001 U or more, preferably 0.002 to 500 U, more preferably 0.005 to 50 U in terms of enzyme activity per gram of the raw material cereal flour. Further, the glucose oxidase is added in 0.00006 to 3 U per unit of α-glucosidase. Note that the enzyme activity of the glucose oxidase is quantified as follows. The glucose oxidase is allowed to act on the substrate glucose in the presence of oxygen to produce hydrogen peroxide, which is then acted upon by peroxidase in the presence of aminoantipyrine and phenol to produce a quinonimine dye. The color of the quinonimine dye is then measured and quantified at wavelength 500 nm. The amount of enzyme required to oxidize 1 µmol of glucose in 1 min is defined as 1 U (unit).

In the present invention, the transglutaminase is add in an appropriate range of 0.0001 to 100 U, preferably 0.0001 to 10 U in terms of enzyme activity per gram of the cereal flour. Further, it is desirable that the transglutaminase be added in 0.0000001 to 1 U, preferably 0.000001 to 0.1 U per unit of α-glucosidase. Note that the enzyme activity of the transglutaminase is measured as follows. The hydroxamic acid produced by a reaction using benzyloxycarbonyl-L-glutaminyl-glycine and hydroxylamine as the substrates is used to form an iron complex in the presence of trichloroacetic acid, and the absorbance at 525 nm is measured. The amount of the hydroxamic acid is then determined from a standard curve, and the activity is calculated. The amount of enzyme that produces 1 µmol of hydroxamic acid in 1 min at 37°C, pH 6.0 is defined as 1 U (unit).

Again, the amounts of enzymes added to produce noodles by the actions of α-glucosidase and glucose oxidase, or by the actions of α-glucosidase, glucose oxidase, and transglutaminase are such that the glucose oxidase is used in an appropriate range of 0.00006 to 3 U in terms of enzyme activity (units) per unit of α-glucosidase, and that the transglutaminase is used in an appropriate range of 0.0000001 to 1 U, preferably 0.000001 to 0.1 U in terms of enzyme activity per unit of α-glucosidase. For pasta, the glucose oxidase is added particularly preferably in 0.0006 to 3 U per unit of α-glucosidase, and the transglutaminase is added particularly preferably in 0.000001 to 0.1 U per unit of α-glucosidase. For udon, the glucose oxidase is added particularly preferably in 0.00006 to 0.3 U per unit of α-glucosidase, and the transglutaminase is added particularly preferably in 0.000001 to 0.1 U per unit of α-glucosidase. With the foregoing ranges of the addition raio of the enzymes, a desirable texture that satisfies both stickiness and strong elasticity can be obtained, and the time-dependent quality deterioration of the product noodles can be suppressed. Further, the turbidity of the cooking liquid from boiling can be reduced.

The reaction time of each enzyme is not particularly limited, as long as the enzyme can act on the substrate substance, and may be very brief or long. In practice, the preferred action time is from 5 min to 24 h. The reaction temperature is not particularly limited either, as long as the enzyme can maintain its activity. In practice, the preferred temperature of action is from 0 to 80°C. In other words, the ordinary noodle manufacturing steps provide a sufficient reaction time.

The enzyme preparation for modifying noodles can be obtained from mixtures of α-glucosidase, glucose oxidase, and transglutaminase with bulking agents such as dextrin, starch, and processed starch; seasonings such as meat extract; proteins such as plant protein, gluten, egg white, gelatin, and casein; protein hydrolysate; protein partial hydrolysate, emulsifiers, chelating agents such as citrates and polyphosphates; reducing agents such as glutathione and cysteine; and other food additives such as alginic acid, kansui, dye, acidulant, and flavoring ingredient. The enzyme preparation used in the present invention may be in the form of a liquid, a paste, a granule, or a powder. The amount of each enzyme mixed in the enzyme preparation is more than 0% and less than 100%. The mixed amount may be 0% for transglutaminase.

### Best Mode for Carrying Out the Invention

The present invention is described below in more detail based on Examples.

### Example 1

The α-glucosidase preparation "Transglucosidase L" (Amano Enzyme Inc.; hereinafter "AG"), the transglutaminase preparation "Activa" TG (Ajinomoto Co., Inc.; hereinafter, "TG"), and the glucose oxidase preparation Sumizyme PGO (Shin-Nihon Chemical Co. hereinafter, "GO") were added to 2 kg of duram flour DF (Nisshin Flour Milling Inc), and thoroughly mixed. Test groups are shown in Table 1. After adding 540 g of tap water, the raw material mixture was kneaded for 15 min with a kneader "Vacuum mixer VU-2" (kuba Tekkosho) with the kneader speed set to 100. After kneading, noodles were made by extruding the mixture through a 1.8-mm long pasta dice using a pasta machine (vacuum extruder FPV-2; Nippun Engineering Co., Ltd.). The extruded strands were then dried with a drier (constant temperature and humidity vessel LH21-13P; Nagano Science) to obtain dry pasta. The dry pasta was boiled in boiling water for 9 min, refrigerated for 24 hours, and heated with a microwave before performing sensory evaluations. The sensory evaluation was done by four panelists for stickiness, elasticity, hardness, firm center, and ease of bite, using the scales from -2 to 2 with a 0 score for control group. The results are shown in Table 1. Note that "stickiness" is the feel of a noodle clinging to the teeth upon chewing, "elasticity" is the stress exerted back from chewing, specifically the extent of resilience, "hardness" is the stress felt upon biting, "firm center" is the Al dente-like texture being harder inside than outside with a hardness gradient from outside toward the center of the strand, and "ease of bite" is the measure of how easily the teeth bite into and cut the noodles. The scales are 0.5 = difference present, 1 = notable difference, 2 = very notable difference. Because of the importance of having both stickiness and strong elasticity for the texture of pasta, the following denotation was used for the test groups:
"*": Stickiness (scores higher than 0)
"*": Notable elasticity (scores of 1 and higher)
"**": Elasticity with the scores of 1.75 and higher

The noodles were deemed to have both stickiness and strong elasticity when the score for stickiness was higher than 0, and when the score for elasticity was 1 or higher, as above.

**Table 1**

| Test group | U/g (Cereal flour) | | | Enzyme activity ratio | | Sensory evaluation results | | | | | | |
|---|---|---|---|---|---|---|---|---|---|---|---|---|
| | AG | TG | GO | GO/AG | TG/AG | Stickiness | | Elasticity | | Hardness | Firm center | Ease of bite |
| Control | 0 | 0 | 0 | 0 | 0 | 0 | | 0 | | 0 | 0 | 0 |
| 1 | 0 | 0 | 0.924 | 0 | 0 | 0 | | 1.25 | * | 0.75 | 0.25 | 1 |
| 2 | 0 | 0.633 | 0 | 0 | 0 | -0.5 | | 0.25 | | 2 | 0 | 1 |
| 3 | 343.8 | 0 | 0 | 0 | 0 | 0.25 | * | 0 | | -0.25 | 0.25 | -0.5 |
| 4 | 137.5 | 0 | 0.693 | 0.005 | 0 | 1 | * | 1 | * | 0.1 | 0.75 | 0.25 |
| 5 | 68.8 | 0 | 0.924 | 0.0134 | 0 | 2 | * | 1.25 | * | 0.25 | 1.25 | 0.75 |
| 6 | 68.8 | 0 | 1.386 | 0.0201 | 0 | 0.75 | * | 1.5 | * | 0.5 | 0.75 | 1.25 |
| 7 | 68.8 | 0.253 | 0.924 | 0.0134 | 0.0037 | 0.5 | * | 2 | ** | 1.75 | 0.25 | 1.5 |
| 8 | 68.8 | 0.127 | 1.155 | 0.0168 | 0.0018 | 0.75 | * | 1.75 | ** | 1.5 | 1 | 1.25 |

As shown in Table 1, GO imparted strong elasticity, but did not impart any stickiness important for providing a desirable texture for pasta. While AG imparted stickiness, hardly any elasticity was imparted, and hardness lowered. Both stickiness and elasticity were imparted in test groups that used AG and GO in combination, and even stronger elasticity was imparted in test groups that also used TG in combination with AG and GO. The results thus demonstrated that pasta with a desirable texture having both stickiness and strong elasticity could be produced with the combined use of AG and GO, or with the combined use of AG, TG, and GO.

### Example 2

AG, TG, and GO were added to 750 g of the all-purpose flour Suzume (Nisshin Flour Milling Inc.), 250 g of the processed starch Ajisai (Matsutani Chemical Industry Co., Ltd.), and 20 g of the wheat gluten A-Glu G (Glico Foods, Co. , Ltd.), and mixed for 1 min at 100 rpm with a 2-kg vacuum kneader (Ohtake Noodle Machine Mfg., Co., Ltd.). The test groups are shown in Table 2. In Table 2, the cereal flour is all-purpose flour, and does not include processed starch. A 5°C brine prepared by adding 30 g of a common salt to 410 g of tap water was added to the total amount of the raw material mixture, and kneaded with a kneader for 5 min (100 rpm for 2 min, 50 rpm for 3 min). After being kneaded, the mixture was processed into a sheet with noodle making machines (small coarse noodle sheeter, small continuous rolling machine; Tom), combined, and press-rolled. The sheet was matured for 1 hour at room temperature, and cut with a #10 cutting blade. The strands were immediately frozen to obtain frozen uncooked udon. The frozen uncooked udon was boiled in boiling water for 7.5 min, and refrigerated for 24 h before performing sensory evaluations. The sensory evaluation was done by four panelists for stickiness, elasticity, hardness, firm center, and stickiness, using the scales from -2 to 2 with a 0 score for control group. The results are shown in Table 2. Note that "stickiness" is the feel of a noodle clinging to the teeth upon chewing, "elasticity" is the stress exerted back from chewing, specifically the extent of resilience, "hardness" is the stress felt upon biting, "firm center" is the Al dente-like texture being harder inside than outside with a hardness gradient from outside toward the center of the strand, and "stickiness" is the measure of adhesion to the teeth after chewing. The scales are 0. 5 = difference present, 1 = notable difference, 2 = very notable difference. Because of the importance of having both stickiness and strong elasticity for the texture of udon, the following denotation was used for the test groups:
"*": Stickiness (scores higher than 0)
"*": Notable elasticity (scores of 1 and higher)

**Table 2**

| Test group | U/g (Cereal flour) | | | Enzyme activity ratio | | Sensory evaluation results | | | | | | |
|---|---|---|---|---|---|---|---|---|---|---|---|---|
| | AG | TG | GO | GO/AG | TG/AG | Stickiness | | Elasticity | | Hardness | Firm center | Ease of bite |
| Control | | | | | | 0 | | 0 | | 0 | 0 | 0 |
| 1 | | | 0.700 | | | 0 | | 2 | * | 1.5 | 0 | 0 |
| 2 | | 0.383 | | | | -1 | | 0.5 | | 2 | 0 | -1 |
| 3 | 208.3 | | | | | 0.25 | * | 0.25 | | -0.5 | 0.25 | 2 |
| 4 | 166.7 | 0.077 | | | 0.0005 | 1.75 | * | 0.5 | | 0.5 | 1.75 | 1.75 |
| 5 | 166.7 | 0.061 | 0.028 | 0.0002 | 0.0004 | 2 | * | 1 | * | 0.5 | 2 | 1.75 |
| 6 | 166.7 | 0.046 | 0.056 | 0.0003 | 0.0003 | 1.5 | * | 1 | * | 0.75 | 1.5 | 1.5 |
| 7 | 166.7 | 0.031 | 0.084 | 0.0005 | 0.0002 | 1.25 | * | 1.25 | * | 0.75 | 1.25 | 1.25 |
| 8 | 166.7 | 0.015 | 0.112 | 0.0007 | 0.0001 | 1 | * | 1.25 | * | 0.75 | 1 | 1 |
| 9 | 166.7 | | 0.140 | 0.0008 | | 1 | * | 1.25 | * | 0.75 | 0.75 | 0.75 |
| 10 | 166.7 | | 0.056 | 0.0003 | | 1.25 | * | 1 | * | 0.25 | 1 | 1.25 |
| 11 | 83.3 | | 0.420 | 0.0050 | | 0.5 | * | 1.75 | * | 1.25 | 0.5 | 0.5 |
| 12 | 41.7 | | 0.560 | 0.0134 | | 0.25 | * | 1.75 | * | 1.25 | 0.5 | 0.25 |
| 13 | 41.7 | 0.153 | 0.560 | 0.0134 | 0.0037 | 0.25 | * | 2 | * | 2 | 0.25 | 0.25 |
| 14 | 41.7 | 0.153 | 0.280 | 0.0067 | 0.0037 | 0.5 | * | 1.75 | * | 1.75 | 0.25 | 0.5 |
| 15 | 41.7 | 0.153 | 0.140 | 0.0034 | 0.0037 | 0.5 | * | 1.25 | * | 1.25 | 0.5 | 0.5 |
| 16 | 83.3 | 0.077 | 0.560 | 0.0067 | 0.0009 | 0.5 | * | 1.75 | * | 1.75 | 0.25 | 0.5 |
| 17 | 83.3 | 0.077 | 0.280 | 0.0034 | 0.0009 | 0.75 | * | 1.5 | * | 1.5 | 0.5 | 0.75 |
| 18 | 83.3 | 0.077 | 0.140 | 0.0017 | 0.0009 | 0.75 | * | 1.25 | * | 1 | 1 | 0.75 |
| 19 | 166.7 | 0.077 | 0.140 | 0.0008 | 0.0005 | 1 | * | 1.5 | * | 1 | 0.75 | 1.25 |
| 20 | 20.8 | 0.077 | 0.280 | 0.0134 | 0.0037 | 0.25 | * | 1.5 | * | 1.5 | 0.25 | 0.25 |

As shown in Table 2, GO imparted strong elasticity, but did not impart any stickiness important for providing a desirable texture for udon. While AG imparted stickiness, sufficient levels of elasticity were not imparted, and hardness lowered. In test groups in which AG and TG were used in combination, the stickiness was notable, and the elasticity was greater than that obtained with the sole use of AG. However, the levels of elasticity were still not sufficient. Both stickiness and strong elasticity were imparted in test groups in which AG and GO, or AG, TG, and GO were used in combination. The results demonstrated that udon with a desirable texture having both stickiness and strong elasticity could be produced with the combined use of AG and GO, or with the combined use of AG, TG, and GO.

### Example 3

Udon was produced in the test groups of Table 3 from the same raw materials used in Example 2 according to the methods of Example 2, and sensory evaluations were performed. The sensory evaluations were performed for the desirable udon texture satisfying both stickiness and strong elasticity, according to the following criteria: "x" = undesirable, "Δ" = marginally desirable, "O" = desirable, and "○○" = very desirable.

As can be seen in test groups 1 to 9 in Table 3, desirable textures with stickiness and strong elasticity, which means that the results were "Δ" or better were obtained with 0.00003 to 30 U of GO added per unit of AG. Similarly, in test groups 10 to 18, desirable textures were obtained with 1 U or less of TG added per unit of AG. It was also confirmed that desirable textures could be obtained with at least 0.0000001 U. Desirable textures were also obtained in test groups 19 to 27 with 1.5 to 300000 U of AG activity per gram of the raw material cereal flour, and with 0.002 to 500 U of GO activity per gram of the raw material cereal flour. The texture was also desirable in test groups 28 to 35, in which the TG activity per gram of the raw material cereal flour was 100 U or less. It was also confirmed that desirable textures was obtained with at least 0.0001 U. Desirable effects were not obtained in the test groups out of the foregoing ranges, in which the effect of each enzyme was either too weak or too strong, disrupting the overall texture balance.

The results confirmed that desirable textures could be obtained with the combined use of AG and GO, or with the combined use of AG, TG, and GO in noodles, when the amount of AG added per gram of the raw material cereal flour is 1.5 to 300000 U, the amount of GO added per gram of the raw material cereal flour is 0.002 to 500 U, and the amount of TG added per gram of the raw material cereal flour is 0.0001 to 100 U and when the amount of GO added per unit of AG is 0.00003 to 30 U and the amount of TG added per unit of AG is 0.0000001 to 1 U.

**Table 3**

| Test group | U/g (Cereal flour) | | | Enzyme activity ratio | | Sensory evaluation results |
|---|---|---|---|---|---|---|
| | AG | TG | GO | GO/AG | TG/AG | |
| Control | 0 | 0 | 0 | 0 | 0 | × |
| 1 | 167 | 0 | 0.0005 | 0.000003 | 0 | × |
| 2 | 167 | 0 | 0.005 | 0.00003 | 0 | Δ |
| 3 | 167 | 0 | 0.05 | 0.0003 | 0 | ○ |
| 4 | 167 | 0 | 0.5 | 0.003 | 0 | ○○ |
| 5 | 17 | 0 | 0.5 | 0.03 | 0 | ○○ |
| 6 | 1.7 | 0 | 0.5 | 0.3 | 0 | ○ |
| 7 | 1.7 | 0 | 5 | 3 | 0 | ○ |
| 8 | 1.7 | 0 | 50 | 30 | 0 | Δ |
| 9 | 1.7 | 0 | 500 | 300 | 0 | × |
| 10 | 16667 | 0.0015 | 50 | 0.003 | 0.0000001 | ○ |
| 11 | 1667 | 0.0015 | 5 | 0.003 | 0.000001 | ○ |
| 12 | 167 | 0.0015 | 0.5 | 0.003 | 0.00001 | ○ |
| 13 | 167 | 0.015 | 0.5 | 0.003 | 0.0001 | ○○ |
| 14 | 167 | 0.15 | 0.5 | 0.003 | 0.001 | ○○ |
| 15 | 167 | 1.5 | 0.5 | 0.003 | 0.01 | ○○ |
| 16 | 167 | 15 | 0.5 | 0.003 | 0.1 | ○ |
| 17 | 17 | 15 | 0.05 | 0.003 | 1 | Δ |
| 18 | 17 | 160 | 0.05 | 0.003 | 10 | × |
| 19 | 600000 | 0 | 1000 | 0.0017 | 0 | × |
| 20 | 300000 | 0 | 500 | 0.0017 | 0 | Δ |
| 21 | 30000 | 0 | 50 | 0.0017 | 0 | ○ |
| 22 | 3000 | 0 | 5 | 0.0017 | 0 | ○ |
| 23 | 300 | 0 | 0.5 | 0.0017 | 0 | ○○ |
| 24 | 30 | 0 | 0.05 | 0.0017 | 0 | ○ |
| 25 | 3 | 0 | 0.005 | 0.0017 | 0 | ○ |
| 26 | 1.5 | 0 | 0.002 | 0.0017 | 0 | Δ |
| 27 | 0.3 | 0 | 0.0005 | 0.0017 | 0 | × |
| 28 | 600000 | 200 | 1000 | 0.0017 | 0.0003 | × |
| 29 | 300000 | 100 | 500 | 0.0017 | 0.0003 | Δ |
| 30 | 30000 | 10 | 50 | 0.0017 | 0.0003 | ○ |
| 31 | 3000 | 1 | 5 | 0.0017 | 0.0003 | ○ |
| 32 | 300 | 0.1 | 0.5 | 0.0017 | 0.0003 | ○○ |
| 33 | 30 | 0.01 | 0.05 | 0.0017 | 0.0003 | ○ |
| 34 | 3 | 0.001 | 0.005 | 0.0017 | 0.0003 | ○ |
| 35 | 3 | 0.0001 | 0.005 | 0.0017 | 0.00003 | ○ |

### Example 4

AG, TG, and GO were added to 1,000 g of the all-purpose flour Shirotsubaki (Nisshin Flour Milling Inc.), and 1 g of the gardenia dye Yellow Color TH-G (T. Hasegawa Co., Ltd.), and mixed for 1 min at 100 rpm with a 2kg-vacuum kneader (Ohtake Noodle Machine Mfg., Co., Ltd.). The test groups are shown in Table 4. A 5°C solution prepared by adding 5 g of a common salt and 10 g of an alkaline preparation (powdery kansui A; Nippon-Colloid) to 420 g of tap water was then added to the total amount of the raw material mixture, and the mixture was kneaded with a kneader for 3.5 min (100 rpm for 2 min, 50 rpm for 1. 5 min). After being kneaded, the mixture was formed into a sheet using noodle making machines (small coarse noodle sheeter, small continuous rolling machine; Tom), combined, and press-rolled. The sheet was matured for 1 hour at room temperature, and cut with a #18 cutting blade. The strands were immediately frozen to obtain frozen uncooked Chinese-style noodles. The frozen Chinese-style noodles were boiled in boiling water for 2.5 min, and refrigerated for 24 hours before performing sensory evaluations for cold Chinese-style noodles. The sensory evaluation was done by four panelists for stickiness and elasticity, using the scales from -2 to 2 with a 0 score for control group. The results are shown in Table 4. The scales are 0. 5 = difference present, 1 = notable difference, 2 = very notable difference. Because of the importance of having both stickiness and strong elasticity for the texture of Chinese-style noodles, the following denotation was used for the test groups:
"*": Stickiness (scores higher than 0)
"*": Notable elasticity (scores of 1 and higher)

**Table 4**

| Test group | U/g (Cereal flour) | | | Enzyme activity ratio | | Sensory evaluation results | | | |
|---|---|---|---|---|---|---|---|---|---|
| | AG | TG | GO | GO/AG | TG/AG | Stickiness | | Elasticity | |
| Control | 0 | 0 | 0 | 0 | 0 | 0 | | 0 | |
| 1 | 208 | 0 | 0 | 0 | 0 | 0.5 | * | 0.25 | |
| 2 | 0 | 0.383 | 0 | 0 | 0 | -1 | | 0.5 | |
| 3 | 0 | 0 | 0.7 | 0 | 0 | 0 | | 2 | * |
| 4 | 188 | 0 | 0.63 | 0.0034 | 0 | 1.75 | * | 1 | * |
| 5 | 167 | 0 | 0.14 | 0.0008 | 0 | 1.5 | * | 1.25 | * |
| 6 | 83 | 0 | 0.42 | 0.005 | 0 | 1 | * | 1.5 | * |
| 7 | 42 | 0 | 0.56 | 0.0134 | 0 | 0.75 | * | 1.75 | * |
| 8 | 42 | 0.153 | 0.56 | 0.0134 | 0.0037 | 0.5 | * | 2 | * |
| 9 | 83 | 0.077 | 0.56 | 0.0067 | 0.0009 | 0.75 | * | 1.75 | * |
| 10 | 175 | 0.077 | 0 | 0 | 0.0004 | 1 | * | 0.5 | |

As shaown in Table 4, the sole addition of GO imparted strong elasticity by the action of GO, but did not impart any stickiness important for providing a desirable texture for Chinese-style noodles. The sole addition of AG imparted stickiness by the action of AG, but did not impart sufficient levels of elasticity. Both stickiness and strong elasticity were imparted in test groups in which AG and GO, or AG, TG, and GO were used in combination. The results demonstrated that Chinese-style noodles with a desirable texture having both stickiness and strong elasticity could be produced with the combined use of AG and GO, or with the combined use of AG, TG, and GO.

### Example 5

Frozen uncooked Chinese-style noodles were obtained in the test groups of Table 5 according to the methods of Example 4. The frozen uncooked Chinese-style noodles were gently loosened, steamed for 7 min, and stir-fried for 30 sec with a source to obtain stir-fried noodles. The stir-fried noodles were refrigerated for 24 hours, and heated in a microwave before performing sensory evaluations. The sensory evaluation was done by four panelists for stickiness and elasticity, using the scales from -2 to 2 with a 0 score for control group. The results are shown in Table 5. The scales are 0. 5 = difference present, 1 = notable difference, 2 = very notable difference. Because of the importance of having both stickiness and strong elasticity for the texture of stir-fried noodles, the following denotation was used for the test groups :
"*": Stickiness (scores higher than 0)
"*": Notable elasticity (scores of 1 and higher)

As shown in Table 5, the sole addition of GO imparted strong elasticity by the action of GO, but did not impart any stickiness important for providing a desirable texture for stir-fried noodles. The sole addition of AG imparted stickiness and elasticity by the action of AG, but the levels of elasticity were slightly weak. Both stickiness and strong elasticity were imparted in test groups in which AG and GO, or AG, TG, and GO were used in combination. The results demonstrated that stir-fried noodles with a desirable texture having both stickiness and strong elasticity could be produced with the combined use of AG and GO, or with the combined use of AG, TG, and GO.

**Table 5**

| Test group | U/g (Cereal flour) | | | Enzyme activity ratio | | Sensory evaluation results | | | |
|---|---|---|---|---|---|---|---|---|---|
| | AG | TG | GO | GO/AG | TG/AG | Stickiness | | Elasticity | |
| Control | 0 | 0 | 0 | 0 | 0 | 0 | | 0 | |
| 1 | 208 | 0 | 0 | 0 | 0 | 0.5 | * | 0.25 | |
| 2 | 0 | 0.383 | 0 | 0 | 0 | -1 | | 0.25 | |
| 3 | 0 | 0 | 0.7 | 0 | 0 | 0 | | 2 | * |
| 4 | 188 | 0 | 0.63 | 0.0034 | 0 | 2 | * | 1 | * |
| 5 | 167 | 0 | 0.14 | 0.0008 | 0 | 2 | * | 1.25 | * |
| 6 | 83 | 0 | 0.42 | 0.005 | 0 | 1.5 | * | 1.5 | * |
| 7 | 42 | 0 | 0.56 | 0.0134 | 0 | 1 | * | 1.75 | * |
| 8 | 42 | 0.153 | 0.56 | 0.0134 | 0.0037 | 0.75 | * | 2 | * |
| 9 | 83 | 0.077 | 0.56 | 0.0067 | 0.0009 | 1 | * | 1.75 | * |
| 10 | 175 | 0.077 | 0 | 0 | 0.0004 | 1.25 | * | 0.75 | |

### Example 6

AG, TG, and GO were added to 500 g of buckwheat flour Heiwa (Hokuto Flour Milling Co., Ltd.) and 500 g of hard flour Seikei (Nissin Seifun), and mixed for 1 min at 100 rpm with a 2-kg vacuum kneader (Ohtake Noodle Machine Mfg. , Co., Ltd.). The test groups are shown in Table 6. In Table 6, the cereal flour means both buckwheat flour and hard flour. A 5°C brine prepared by adding 15 g of a common salt to 350 g of tap water was added to the total amount of the raw material mixture, and the mixture was kneaded with a kneader for 5 min (100 rpm for 2 min, 50 rpm for 3 min). After being kneaded, the mixture was formed into a sheet with noodle making machines (small coarse noodle sheeter, small continuous rolling machine; Tom), combined, and press-rolled. The sheet was matured for 1 hour at room temperature, and cut with a #18 cutting blade. The strands were immediately frozen to obtain frozen uncooked buckwheat noodle. The frozen uncooked buckwheat noodle was boiled in boiling water for 2.5 min, and refrigerated for 24 hours before performing sensory evaluations. The sensory evaluation was done by four panelists for stickiness and elasticity, using the scales from -2 to 2 with a 0 score for control group. The results are shown in Table 6. Despite that important textures for buckwheat noodle generally include hardness, elasticity, and ease of bite, because stickiness and elasticity are considered important in varieties such as country-style buckwheat noodle, the following denotation was used for the test groups in the invention:
"*": Stickiness (scores higher than 0)
"*": Notable elasticity (scores of 1 and higher)

**Table 6**

| Test group | U/g (Cereal flour) | | | Enzyme activity ratio | | Sensory evaluation results | | | |
|---|---|---|---|---|---|---|---|---|---|
| | AG | TG | GO | GO/AG | TG/AG | Stickiness | | Elasticity | |
| Control | 0 | 0 | 0 | 0 | 0 | 0 | | 0 | |
| 1 | 208 | 0 | 0 | 0 | 0 | 0.5 | * | 0.25 | |
| 2 | 0 | 0.383 | 0 | 0 | 0 | -1 | | 0.25 | |
| 3 | 0 | 0 | 0.7 | 0 | 0 | 0 | | 2 | * |
| 4 | 42 | 0.153 | 0.56 | 0.0134 | 0.0037 | 0.5 | * | 2 | * |
| 5 | 42 | 0.153 | 0 | 0 | 0.0037 | 0.75 | * | 0.5 | |
| 6 | 42 | 0 | 0.56 | 0.0134 | 0 | 1 | * | 1.75 | * |
| 7 | 0 | 0.153 | 0.56 | 0 | 0 | -0.5 | | 1.5 | * |

As shown in Table 6, the sole addition of GO imparted strong elasticity by the action of GO, but did not impart any stickiness. The sole addition of AG imparted stickiness and elasticity by the action of AG, but the levels of elasticity were slightly weak. Both stickiness and strong elasticity were imparted in test groups in which AG and GO, or AG, TG, and GO were used in combination. The results demonstrated that buckwheat noodle with a desirable texture having both stickiness and strong elasticity could be produced with the combined use of AG and GO, or with the combined use of AG, TG, and GO.

### Comparative Example

JP-A-6-296467 describes improving chewiness with the combined use of glucose oxidase, amylase (AM), and glucoamylase (GA). For comparison with the present invention, udon was prepared using the same materials and methods used in Example 2 and a sensory evaluation was done. The sensory evaluation was done by four panelists for stickiness and elasticity, using the scales from -2 to 2 with a 0 score for control group. The results are shown in Table 7. The scales are 0. 5 = difference present, 1 = notable difference, 2 = very notable difference. Because of the importance of having both stickiness and strong elasticity for the texture of udon, the following denotation was used for the test groups:
"*": Stickiness (scores higher than 0)
"*": Notable elasticity (scores of 1 and higher)

The test groups are shown in Table 7. Note that Amylase AD Amano 1 and Gluczyme AF6 (both available from Amano Enzyme Inc.) were used as α-amylase and glucoamylase, respectively. The amounts of α-amylase, glucoamylase, and GO in the test groups 1 to 3 in Table 7 were set according to Example 1, Comparative Example 2, and Comparative Example 3 of JP-A-6-296467.

**Table 7**

| Test group | U/g (Cereal flour) | | | | | Enzyme activity ratio | | Sensory evaluation results | | | |
|---|---|---|---|---|---|---|---|---|---|---|---|
| | AM | GA | GO | AG | TG | GO/AG | TG/AG | Stickiness | | Elasticity | |
| Control | 0 | 0 | 0 | 0 | 0 | 0 | 0 | 0 | | 0 | |
| 1 | 0.5 | 0 | 0 | 0 | 0 | 0 | 0 | 0.25 | * | -0.25 | |
| 2 | 0.4 | 0.01 | 0 | 0 | 0 | 0 | 0 | 0.25 | * | -0.25 | |
| 3 | 0.3 | 0.01 | 0.2 | 0 | 0 | 0 | 0 | 0.75 | * | 0.5 | |
| 4 | 0 | 0 | 0.2 | 0 | 0 | 0 | 0 | 0 | | 2 | * |
| 5 | 0 | 0 | 0.2 | 300 | 0 | 0.0007 | 0 | 1.5 | * | 1.5 | * |
| 6 | 0 | 0 | 0.2 | 300 | 0.1 | 0.0007 | 0.0003 | 1 | * | 2 | * |

As shown in Table 7, the addition of amylase, or the addition of amylase and glucoamylase imparted stickiness; however, these additions tended to weaken elasticity slightly. The strong levels of elasticity as obtained in the present invention could not be obtained with the combined use of amylase, glucoamylase, and GO. The texture obtained from the use of amylase was mainly characterized by heavy, gluey stickiness, largely different from the feel of stickiness, which is the feel of the noodle clinging to the teeth upon chewing, as defined in the present invention. Further, the use of amylase had a pronounced effect on softness, and had the tendency to make the elasticity slightly weak. Additional tests confirmed that this tendency became more pronounced as the reaction time of the enzyme increased in the producing steps. This greatly differs from the present invention. The combined use of GO and AG, or the combined use of GO, AG, and TG provided very desirable textures that satisfied both stickiness and strong elasticity. These results demonstrated that the present invention, capable of imparting stickiness and strong elasticity with the combined use of AG and GO, or AG, GO, and TG without imparting stickiness or softness, is greatly different from JP-A-6-296467, and represents a useful finding.

### Industrial Applicability

The present invention can improve noodle qualities, and is therefore highly useful in the field of food.

## Claims

1. A method for producing a noodle having improved stickiness and elasticity with α-glucosidase and glucose oxidase, wherein the α-glucosidase is used in 1.5 to 300,000 U per gram of a raw material cereal flour, and wherein the glucose oxidase is used in 0.002 to 500 U per gram of the raw material cereal flour and in 0.00006 to 3 U per unit of the α-glucosidase.

2. The method according to claim 1, wherein the α-glucosidase is used in 3 to 15,000 U per gram of the raw material cereal flour, and wherein the glucose oxidase is used in 0.005 to 50 U per gram of the raw material cereal flour.

3. The method according to claim 1, wherein transglutaminase is further used.

4. The method according to claim 3, wherein the transglutaminase is used in 0.0001 to 100 U per gram of the raw material cereal flour.

5. The method according to claim 3, wherein the transglutaminase is used in 0.0001 to 10 U per gram of the raw material cereal flour.

6. The method according to claim 3, wherein the transglutaminase is used in 0.0000001 to 1 U per unit of the α-glucosidase.

7. The method according to claim 3, wherein the glucose oxidase is used in 0.00006 to 3 U per unit of the α-glucosidase, and wherein the transglutaminase is added in 0.000001 to 0.1 U per unit of the α-glucosidase.

8. The method according to claim 1, wherein the noodle is any one of pasta, udon, Chinese-style noodle, stir-fried noodle and buckwheat noodle.

9. The use of an enzyme preparation for modifying a noodle to have improved stickiness and elasticity, wherein the enzyme preparation contains α-glucosidase and glucose oxidase as active ingredients, wherein the content of the glucose oxidase is 0.00006 to 3 U per unit of the α-glucosidase, and wherein the α-glucosidase is used in 1.5 to 300,000 U per gram of a raw material cereal flour, and the glucose oxidase is used in 0.002 to 500 U per gram of the raw material cereal flour.

10. The use according to claim 9, wherein the enzyme preparation further contains transglutaminase as an active ingredient.

11. The use according to claim 10, wherein the content of the transglutaminase is 0.0000001 to 1 U per unit of the α-glucosidase.

12. The use according to claim 10, wherein the content of the transglutaminase is 0.000001 to 0.1 U per unit of the α-glucosidase.

## Patentansprüche

1. Verfahren zum Herstellen einer Nudel mit verbesserter Klebrigkeit und Elastizität mit α-Glukosidase und Glukoseoxidase, wobei die α-Glukosidase in einer Menge von 1,5 bis 300000 U pro Gramm eines Ausgangsmaterialgetreidemehls eingesetzt wird, und wobei die Glukoseoxidase in einer Menge von 0,002 bis 500 U pro Gramm des Ausgangsmaterialgetreidemehls und in einer Menge von 0,00006 bis 3 U pro Einheit der α-Glukosidase eingesetzt wird.

2. Verfahren nach Anspruch 1, wobei die α-Glukosidase in einer Menge von 3 bis 15000 U pro Gramm des Ausgangsmaterialgetreidemehls eingesetzt wird, und wobei die Glukoseoxidase in einer Menge von 0,005 bis 50 U pro Gramm des Ausgangsmaterialgetreidemehls eingesetzt wird.

3. Verfahren nach Anspruch 1, wobei außerdem Transglutaminase eingesetzt wird.

4. Verfahren nach Anspruch 3, wobei die Transglutaminase in einer Menge von 0,0001 bis 100 U pro Gramm des Ausgangsmaterialgetreidemehls eingesetzt wird.

5. Verfahren nach Anspruch 3, wobei die Transglutaminase in einer Menge von 0,0001 bis 10 U pro Gramm des Ausgangsmaterialgetreidemehls eingesetzt wird.

6. Verfahren nach Anspruch 3, wobei die Transglutaminase in einer Menge von 0,0000001 bis 1 U pro Einheit der α-Glukosidase eingesetzt wird.

7. Verfahren nach Anspruch 3, wobei die Glukoseoxidase in einer Menge von 0,00006 bis 3 U pro Einheit der α-Glukosidase eingesetzt wird, und wobei die Transglutaminase in einer Menge von 0,000001 bis 0,1 U pro Einheit der α-Glukosidase zugesetzt wird.

8. Verfahren nach Anspruch 1, wobei die Nudel beliebig unter Pasta, Udon, Nudeln nach chinesischer Art, gebratenen Nudeln und Buchweizennudeln ausgewählt sind.

9. Verwendung einer Enzymzubereitung zum Modifizieren einer Nudel mit verbesserter Klebrigkeit und Elastizität, wobei die Enzymzubereitung α-Glukosidase und Glukoseoxidase als aktive Bestandteile enthält, wobei der Gehalt der Glukoseoxidase 0,00006 bis 3 U pro Einheit der α-Glukosidase ist, und wobei die α-Glukosidase in einer Menge von 1,5 bis 300000 U pro Gramm eines Ausgangsmaterialgetreidemehls eingesetzt wird und die Glukoseoxidase in einer Menge von 0,002 bis 500 U pro Gramm des Ausgangsmaterialgetreidemehls eingesetzt wird.

10. Verwendung nach Anspruch 9, wobei die Enzymzubereitung außerdem Transglutaminase als einen aktiven Bestandteil enthält.

11. Verwendung nach Anspruch 10, wobei der Gehalt der Transglutaminase 0,0000001 bis 1 U pro Einheit der α-Glukosidase ist.

12. Verwendung nach Anspruch 10, wobei der Gehalt der Transglutaminase 0,000001 bis 0,1 U pro Einheit der α-Glukosidase ist.

## Revendications

1. Procédé de préparation d'une nouille ayant une capacité à coller et une élasticité améliorées comportant de l'α-glucosidase et de la glucose oxydase, dans lequel l'α-glucosidase est utilisée à raison de 1,5 à 300 000 U par gramme d'une matière première constituée de farine de céréales, et dans lequel la glucose oxydase est utilisée à raison de 0,002 à 500 U par gramme de la matière première constituée de farine de céréales et à raison de 0,00006 à 3 U par unité de l'α-glucosidase.

2. Procédé selon la revendication 1, dans lequel l'α-glucosidase est utilisée à raison de 3 à 15 000 U par gramme de la matière première constituée de farine de céréales, et dans lequel la glucose oxydase est utilisée à raison de 0,005 à 50 U par gramme de la matière première constituée de farine de céréales.

3. Procédé selon la revendication 1, dans lequel une transglutaminase est également utilisée.

4. Procédé selon la revendication 3, dans lequel la transglutaminase est utilisée à raison de 0,0001 à 100 U par gramme de la matière première constituée de farine de céréales.

5. Procédé selon la revendication 3, dans lequel la transglutaminase est utilisée à raison de 0,0001 à 10 U par gramme de la matière première constituée de farine de céréales.

6. Procédé selon la revendication 3, dans lequel la transglutaminase est utilisée à raison de 0,0000001 à 1 U par unité de l'α-glucosidase.

7. Procédé selon la revendication 3, dans lequel la glucose oxydase est utilisée à raison de 0,00006 à 3 U par unité de l'α-glucosidase, et dans lequel la transglutaminase est ajoutée en une quantité de 0,000001 à 0,1 U par unité de l'α-glucosidase.

8. Procédé selon la revendication 1, dans lequel la nouille est l'une quelconque parmi une pâte, une nouille épaisse, une nouille chinoise, une nouille frite et une nouille au sarrasin.

9. Utilisation d'une préparation enzymatique destinée à modifier une nouille pour qu'elle ait une capacité à coller et une élasticité améliorées, dans laquelle la préparation enzymatique contient de l'α-glucosidase et de la glucose oxydase en tant que substances actives, dans laquelle la teneur de la glucose oxydase est de 0,00006 à 3 U par unité de l'α-glucosidase, et dans laquelle l'α-glucosidase est utilisée à raison de 1,5 à 300 000 U par gramme d'une matière première constituée de farine de céréales, et la glucose oxydase est utilisée à raison de 0,002 à 500 U par gramme de la matière première constituée de farine de céréales.

10. Utilisation selon la revendication 9, dans laquelle la préparation enzymatique contient en outre de la transglutaminase en tant que substance active.

11. Utilisation selon la revendication 10, dans laquelle la teneur de la transglutaminase est de 0,0000001 à 1 U par unité de l'α-glucosidase.

12. Utilisation selon la revendication 10, dans laquelle la teneur de la transglutaminase est de 0,000001 à 0,1 U par unité de l'α-glucosidase.
